(19) Europäisches Patentamt
European Patent Office
Office européen des brevets

(11) Veröffentlichungsnummer: **0 081 666**
**A1**

(12) # EUROPÄISCHE PATENTANMELDUNG

(21) Anmeldenummer: **82110139.1**

(22) Anmeldetag: **04.11.82**

(51) Int. Cl.³: **C 07 C 33/46**, C 07 C 29/40

(30) Priorität: **14.11.81 DE 3145286**

(43) Veröffentlichungstag der Anmeldung: **22.06.83**
**Patentblatt 83/25**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Hoppmann, Angela, Dr., Morgengraben 4, D-5000 Koeln 80 (DE)**
Erfinder: **Jautelat, Manfred, Dr., Muellersbaum 28, D-5093 Burscheid (DE)**
Erfinder: **Arlt, Dieter, Dr. Prof., Rybniker Strasse 2, D-5000 Koeln 80 (DE)**

(54) **Verfahren zur Herstellung von Pentafluorbenzylalkohol.**

(57) Die vorliegende Erfindung betrifft ein neues zur Herstellung von Pentafluorbenzylalkohol der Formel I

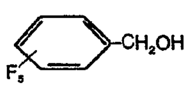

(I)

indem man Chlorpentafluorbenzol der Formel II

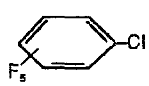

(II)

mit Magnesiumspänen zur Grignard-Verbindung der Formel III

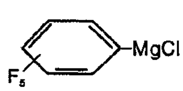

(III)

und anschließend mit gasförmigem Formaldehyd umsetzt, das dadurch gekennzeichnet ist, daß die Reaktion in einem Gemisch aus aromatischen Kohlenwasserstoffen und Ethern bei Temperaturen zwischen 0 und 25 °C durchgeführt wird.



ACTORUM AG

0081666

BAYER AKTIENGESELLSCHAFT 5090 Leverkusen, Bayerwerk

Zentralbereich
Patente, Marken und Lizenzen Rt/Kü-c
IVa / ZP

## Verfahren zur Herstellung von Pentafluorbenzylalkohol

Die vorliegende Erfindung betrifft ein neues Verfahren zur Herstellung von Pentafluorbenzylalkohol.

Es ist bekannt, Pentafluorbenzylalkohol durch Hydrolyse des Pentafluorbenzylchlorids (A.K. Barbour et. al., J. Chem. Soc. 1961, 808) herzustellen. Die Ausgangsverbindung wird durch Chlorierung von Pentafluortoluol im UV-Licht hergestellt. Pentafluortoluol wird nach Literatur durch eine mehrstufige Synthese hergestellt (A. Cairncross, W.A. Sheppard, J. Amer. Chem. Soc. 90, 2186 (1968)). Dieses Verfahren hat den Nachteil, über fünf Stufen zu verlaufen.

Es ist bekannt, Pentafluorbenzylalkohol durch Reduktion der Pentafluorbenzoesäure nach literaturbekannter Methode mit $LiAlH_4$ herzustellen. Pentafluorbenzoesäure wird durch zweimalige Chlorierung von Pentafluortoluol und anschließende Hydrolyse (A.K. Barbour et al., J. Chem. Soc. 1961, 808) oder durch Umsetzung einer metallorganischen Verbindung mit Kohlendioxid (C. Tamborski, E.J. Soloski, J. Org. Chem. 31, 743 (1966) und 31, 4229 (1966)) hergestellt. Diese Verfahren sind mehrstufig,

und es muß im letzten Reaktionsschritt das leicht entzündliche und teure $LiAlH_4$ eingesetzt werden.

Es ist ebenfalls bekannt, Pentafluorbenzylalkohol durch Reduktion von Penatfluorbenzaldehyd mit $LiAlH_4$ (A.K. Barbour, M.W. Buxton, P. L. Coe, R. Stephens und J.C. Tatlow, J. Chem. Soc. 1961, 808 und BE 603597) herzustellen. Der Aldehyd kann durch Umsetzung des Pentafluormagnesiumchlorids mit N-Methyl-formanilid (N.N. Vorozhtsov et al., Dokl. Akad. Nauk SSSR 159, 125 (1964), C.A. 62, 4045 (1965)) hergestellt werden. Dieses Verfahren ist mehrstufig und hat außerdem den Nachteil, daß teures, leicht entzündendes $LiAlH_4$ eingesetzt werden muß.

Es ist bekannt, Pentafluorbenzylalkohol durch Umsetzung von Pentafluormagnesiumchlorid mit gasförmigen Formaldehyd in Diethylether herzustellen (V.P. Molosnova., V.A. Barkhash, N.N. Vorozhtsov, Zh. Obshch. Khim. 39, 1774 (1969); J. Gen. Chem. USSR 39, 1737 (1969)). Dieses Verfahren hat den Nachteil, daß als Lösungsmittel der leicht brennbare Diethylether und als Hilfsreagens das teure und zudem krebserregende Dibromethan eingesetzt werden. Damit kommt dieses Verfahren für den technischen Einsatz nicht in Betracht.

Es ist bekannt (DAS 1 223 383, DAS 1 284 423 und A.E. Jukes und H. Gilman, J. Organometallic Chem. 17, 145 (1969)), daß Pentafluormagnesiumchlorid in Tetrahydrofuran vorzugsweise bei -10°C hergestellt werden kann.

Dieses Verfahren hat den Nachteil, daß teures, brennbares Tetrahydrofuran als Lösungsmittel verwendet wird. Es sind tiefe Reaktionstemperaturen erforderlich, da die Grignard-Verbindung sich bei höheren Temperaturen teilweise zersetzt.

Es wurde nun gefunden, daß man Pentafluorbenzylalkohol der Formel I

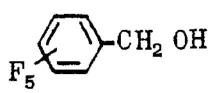 (I)

in sehr guten Ausbeuten in einem Eintopfverfahren erhält, indem man Chlorpentafluorbenzol der Formel II

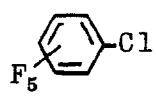 (II)

mit Magnesiumspänen zur Grignard-Verbindung der Formel III

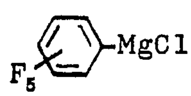 (III)

und anschließend mit gasförmigen Formaldehyd umsetzt, das dadurch gekennzeichnet ist, daß als Lösungsmittel ein Gemisch aus aromatischen Kohlenwasserstoffen und Ethern im Temperaturbereich von 0 bis 25°C verwendet werden.

Es ist als überraschend zu bezeichnen, daß der Pentafluorbenzylalkohl in hoher Ausbeute bei Verwendung eines aromatischen Kohlenwasserstoffs mit einem Ether als Komplexbildner im Temperaturbereich von 0 bis 25°C erhalten wird, da bekannt ist, daß sich die Grignard-Verbindung (III) in reinem THF bei 28°C rasch zersetzt (A.E. Jukes und H. Gilman, J. Organometallic Chem. 17, 145 (1969)).

Das erfindungsgemäße Verfahren weist eine Reihe von Vorteilen auf. Es wird nur die zur Komplexierung der Grignard-Verbindung benötigte Menge Ether gebraucht, zur weiteren Verdünnung kann der billigere und ungefährlichere Kohlenwasserstoff eingesetzt werden. Es ist keine aufwendige Kühlung notwendig, da im Temperaturbereich 0 bis 25°C gearbeitet wird. Die Umsetzung zur Grignard-Verbindung (III) geht rasch.

Pentafluorchlorbenzol ist gut zugänglich. Als Verdünnungsmittel für das erfindungsgemäße Verfahren kommen alle aromatischen Kohlenwasserstoffe wie Benzol, Toluol, Xylol infrage, vorzugsweise Toluol.

Als Komplexierungsmittel für die Grignard-Verbindung kommen alle Ether wie Diethylether, THF, Dioxan, Dimethoxyethan infrage, vorzugsweise THF.

Der Ether wird in 1- bis 10-fachem Überschuß (zum eingesetzten Halogenid) eingesetzt, vorzugsweise in 3- bis 5-fachem Überschuß.

Das Magnesium wird in Form von handelsüblichen Spänen eingesetzt.

Die Umsetzung zur Grignard-Verbindung erfolgt im Temperaturbereich von 0 bis +25°C, vorzugsweise von 10 bis 20°C . Der gasförmige Formaldehyd wird in üblicher Weise erzeugt, z.B. durch Depolymerisation von wasserfreiem Paraformaldehyd in wasserfreien 1,2-Dichlorbenzol, und mit einem $N_2$-Strom übergeleitet. Die Umsetzung der Grignard-Verbindung mit dem gasförmigen Formaldehyd erfolgt bei 0 bis 25°C, vorzugsweise bei 10 bis 20°C. Die Aufarbeitung erfolgt durch Zugabe einer wäßrigen Säure, vorzugsweise Schwefelsäure, Extraktion mit einem mit Wasser nicht mischbaren Lösungsmittel, vorzugsweise Toluol, Waschen der organischen Phase mit gesättigter Kochsalzlösung, Trocknen der organischen Phase und Abdestillieren des Verdünnungsmittels. Das erhaltene Produkt der Formel (I) kann, wenn notwendig, durch Destillation gereinigt werden.

Das nachfolgende Beispiel erläutert das erfindungsgemäße Verfahren.

Beispiel

In einer trockenen Apparatur werden unter Stickstoff 7 g (0,3 Mol) Magnesiumspäne, die mit Jod angeätzt sind, und ein Teil des Gemisches von 50 ml wasserfreiem Toluol und 43 g (0,6 Mol) wasserfreiem Tetrahydrofuran vorgelegt. Von der insgesamt eingesetzten Menge von 40,4 g (0,2 Mol) Chlorpentafluorbenzol wird ein kleiner Teil zugesetzt, so daß die Reaktion anspringt. Das Gemisch wird durch Kühlen im Temperaturbereich von 10 bis 15°C gehalten. Die restlichen Mengen Lösungsmittelgemisch bzw. Chlorpentafluorbenzol werden parallel in dem Maße zugegeben, daß die Reaktion weiterläuft. Nach beendeter Zugabe werden weitere 20 Minuten bei 15°C nachgerührt, und dann wird in den Reaktionskolben im $N_2$-Storm gasförmiger Formaldehyd eingeleitet, der durch Erhitzen von getrocknetem Paraformaldehyd in wasserfreiem 1,2-Dichlorbenzol auf 150!C erzeugt wird. Man läßt auf Raumtemperatur kommen und setzt zur Aufarbeitung Eis/20 %ige Schwefelsäure zu. Nach mehrfacher Extraktion mit Toluol wird die organische Phase mit Wasser gewaschen, mit Natriumsulfat getrocknet und das Lösungsmittel abdestilliert. Die Destillation des Rohprodukts bei $Kp_{760}$: 185°C liefert 35,6 g Pentafluorbenzylalkohol. Die Ausbeute beträgt 90 % der Theorie.

Patentansprüche

1. Verfahren zur Herstellung von Pentafluorbenzylalkohol der Formel I

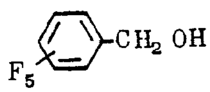

(I)

indem man Chlorpentafluorbenzol der Formel II

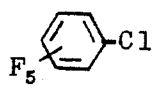

(II)

mit Magnesiumspänen zur Grignard-Verbindung der Formel III

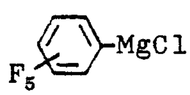

(III)

und anschließend mit gasförmigen Formaldehyd umsetzt, das dadurch gekennzeichnet ist, daß die Reaktion in einem Gemisch aus aromatischen Kohlenwasserstoffen und Ethern bei Temperaturen zwischen 0 und 25°C durchgeführt wird.

Europäisches Patentamt

# EUROPÄISCHER RECHERCHENBERICHT

0081666

Nummer der Anmeldung

EP 82 11 0139

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | Chemical Abstracts Band 71, Nr. 25, 22 Dezember 1969, Columbus, Ohio, USA V.P. MOLOSNOVA et al. "Transformations of decafluoro-alpha-phenylcinnamic acid", Seite 424, Spalte 2,Abstract Nr. 124121a & Zh.Obshch.Khim., Band 39,Nr. 8, 1969 (Cat. A,D) | 1 | C 07 C 33/46<br>C 07 C 29/40 |
| A | DE-B-1 284 423 (I.C.I.)<br>* Anspruch 1 * | 1 | |
| A | GB-A- 985 650 (IMPERIAL SMELTING CORP.)<br>* Anspruch 1 * | 1 | RECHERCHIERTE SACHGEBIETE (Int. Cl. 3)<br>C 07 B 17/00<br>C 07 C 29/40<br>C 07 C 33/46 |

Der vorliegende Recherchenbericht wurde für alle Patentansprüche erstellt.

| Recherchenort | Abschlußdatum der Recherche | Prüfer |
|---|---|---|
| BERLIN | 08-02-1983 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82